# EUROPEAN PATENT APPLICATION

(11) **EP 2 982 968 A1**
(43) Date of publication of application: **10.02.2016**
(21) Application number: 14778556.2
(22) Date of filing: 03.04.2014
(51) Int. Cl.: G01N 21/65, C12M 1/34, C12Q 1/02

(54) **CELL OBSERVATION METHOD, CELL OBSERVATION DEVICE, CELL OBSERVATION PROGRAM, CELL-SHEET MANUFACTURING METHOD, AND CELL-SHEET MANUFACTURING DEVICE**

(30) Priority: 05.04.2013 JP 2013079936
(71) Applicant: Nikon Corporation, Tokyo 108-6290 (JP)
(72) Inventor: FUKUTAKE, Naoki, Tokyo 108-6290 (JP); AIKAWA, Naoshi, Tokyo 108-6290 (JP)
(74) Representative: Hoffmann Eitle
(86) International application number: PCT/JP2014/001936
(87) International publication number: WO 2014/162744

(57) **Abstract**

A cell observation method includes a detection step of detecting radiation light that is radiated from an observation target when the observation target is irradiated with irradiation light; and a determination step of determining whether a cell included in the observation target is alive or dead based on the radiation light. Another cell observation method may include a detection step of detecting radiation light that is radiated from an observation target when the observation target is irradiated with irradiation light; and a detection step of detecting the number of live cells included in the observation target based on the radiation light. Another cell observation method may include an acquisition step of acquiring a spectrum of irradiation light that irradiates an observation target when the observation target is irradiated with the irradiation light; and a detection step of detecting a state of at least one of lipid and protein included in the observation target based on the spectrum.

## Description

### BACKGROUND

### 1. TECHNICAL FIELD

The present invention relates to a cell observation method, a cell observation apparatus, and a cell observation program.

### 2. RELATED ART

A method has been proposed for observing a cell without damaging the cell, as shown in Patent Document 1, for example.
Patent Document 1: Japanese Patent Application Publication No. 2012-143231

The judgment between a live cell and a dead cell is made by observing the different behaviors of the cells, and therefore it is impossible to observe a cellular composition of a sample while maintaining this cellular composition.

### SUMMARY

According to a first aspect of the present invention, provided is a cell observation method comprising a detection step of detecting radiation light that is radiated from an observation target when the observation target is irradiated with irradiation light; and a determination step of determining whether a cell included in the observation target is alive or dead based on the radiation light.

According to a second aspect of the present invention, provided is a cell observation method comprising a detection step of detecting radiation light that is radiated from an observation target when the observation target is irradiated with irradiation light; and a detection step of detecting the number of live cells included in the observation target based on the radiation light.

According to a third aspect of the present invention, provided is a cell observation method comprising an acquisition step of acquiring a spectrum of irradiation light that irradiates an observation target when the observation target is irradiated with the irradiation light; and a detection step of detecting a state of at least one of lipid and protein included in the observation target based on the spectrum.

According to a fourth aspect of the present invention, provided is a cell observation method comprising a detection step that includes detecting Raman-scattered light generated from the observation target by irradiating the observation target with one coherent light having one wavelength and another coherent light having another wavelength that has a wavelength difference relative to the wavelength of the one coherent light corresponding to normal mode oscillation of molecules included in a cell of the observation target, and a determination step that includes determining whether a cell included in the observation target is dead or alive based on the Raman-scattered light detected in the detection step.

According to a fifth aspect of the present invention, provided is a cell observation apparatus comprising a detecting section that detects Raman-scattered light generated from the observation target by irradiating the observation target with one coherent light having one wavelength and another coherent light having another wavelength that has a wavelength difference relative to the wavelength of the one coherent light corresponding to normal mode oscillation of molecules included in a cell of the observation target, and a determining section that determines whether a cell included in the observation target is dead or alive based on the Raman-scattered light detected by the detecting section.

According to a sixth aspect of the present invention, provided is a cell observation program that causes a calculator to detect Raman-scattered light generated from the observation target by irradiating the observation target with one coherent light having one wavelength and another coherent light having another wavelength that has a wavelength difference relative to the wavelength of the one coherent light corresponding to normal mode oscillation of molecules included in a cell of the observation target, and determine whether a cell included in the observation target is dead or alive based on the detected Raman-scattered light.

According to a seventh aspect of the present invention, provided is a cell sheet manufacturing method comprising a preparation step of dividing cells to prepare a cell line; a culture step of culturing the cell line in a cell sheet; and a determination step of observing the cells using a cell observation method and determining whether the cells are dead or alive.

According to an eighth aspect of the present invention, provided is a cell sheet manufacturing apparatus comprising a preparing section that divides cells to prepare a cell line; a culturing section that cultures the cell line in a cell sheet; and a determining section that observes the cells using a cell observation method and determines whether the cells are dead or alive.

The summary clause does not necessarily describe all necessary features of the embodiments of the present invention. The present invention may also be a subcombination of the features described above.

### BRIEF DESCRIPTION OF THE DRAWINGS

Fig. 1 is a schematic view of the structure of a laser microscope 100.
Fig. 2 is a block diagram of the control section 162.
Fig. 3 is a schematic view for describing generation of the anti-Stokes Raman-scattered light.
Fig. 4 shows an exemplary flow of a preparation process performed using the laser microscope 100.
Fig. 5 shows a spectrum image extracted from the sample 112.
Fig. 6 shows a detection image obtained from a live cell.
Fig. 7 shows a detection image obtained from a live cell.
Fig. 8 shows a detection image obtained from a dead cell.
Fig. 9 shows a detection image obtained from a dead cell.
Fig. 10 shows a detection image obtained from a dead cell.
Fig. 11 shows an exemplary flow of an observation process performed using the laser microscope 100.
Fig. 12 shows exemplary image processing performed on the detection image.
Fig. 13 shows exemplary image processing performed on the detection image.
Fig. 14 shows exemplary image processing performed on the detection image.
Fig. 15 shows exemplary image processing performed on the detection image.
Fig. 16 shows exemplary image processing performed on the detection image.
Fig. 17 is a schematic view of a three-dimensional structure of a cell in the sample 112.
Fig. 18 is a schematic view of a three-dimensional distribution of a cell in the sample 112.
Fig. 19 shows a combination of a plurality of layers of detection images.
Fig. 20 shows a detection image in the width direction.
Fig. 21 is a schematic view for describing generation of stimulated Raman-scattered light.

### DESCRIPTION OF EXEMPLARY EMBODIMENTS

Hereinafter, some embodiments of the present invention will be described. The embodiments do not limit the invention according to the claims, and all the combinations of the features described in the embodiments are not necessarily essential to means provided by aspects of the invention.

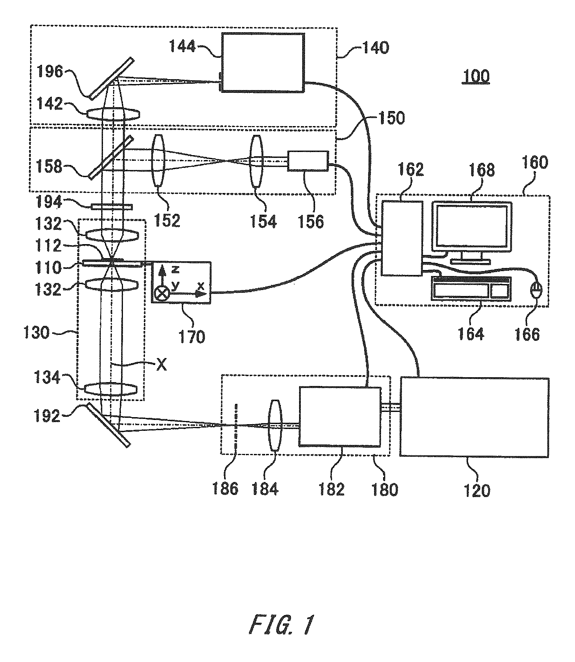

Fig. 1 is a schematic view of the structure of a laser microscope 100. The laser microscope 100 includes a stage 110, a laser apparatus 120, a confocal optical system 130, a first detection system 140, a second detection system 150, a control system 160, a first scanning system 170, and a second scanning system 180.

With the laser microscope 100, a sample 112 placed on the stage 110 is observed. With the laser microscope 100, irradiation light generated by the laser apparatus 120 passes through the confocal optical system 130 to irradiate the sample 112. Emission light from the sample 112 that has been irradiated with the irradiation light is detected by the first detection system 140 or the second detection system 150.

The control system 160 controls the overall operation of the laser microscope 100, according to instructions received from a user. The control system 160 generates output information based on the detection results of the first detection system 140 or the second detection system 150. The output information may be an image that reflects the structure of the sample 112. Furthermore, the output information may be a character sequence or code indicating a determination result that reflects the state of the sample 112.

The first detection system 140 is used together with the first scanning system 170 that displaces the stage 110. The first scanning system 170 includes a stage drive section that moves the stage 110 horizontally. By irradiating the stage on which the sample 112 is mounted with the irradiation light while the stage is moved horizontally by the stage drive section, the first detection system 140 can detect the emission light that is emitted from an observation target region that has a predetermined width extracted as a portion of the sample 112.

The second detection system 150 is used together with the second scanning system 180 that displaces the optical path of the irradiation light irradiating the sample 112. The second detection system 150 includes a galvanic scanner 182 and a scanner lens 184. The galvanic scanner 182 includes a reflective mirror that pivots around two axes having different orientations, and displaces the optical path of the irradiation light incident thereto two-dimensionally in a direction intersecting the optical axis. While the first detection system 140 is being used, the operation of the galvanic scanner 182 of the second scanning system 180 is stopped and the optical axis of the irradiation light is fixed.

The scanner lens 184 creates a focal point on a predetermined primary image surface 186, regardless of the position of the irradiation light radiated from the galvanic scanner 182. In this way, the second detection system 150 can detect the emission light emitted from the observation target region having a predetermined width extracted as a portion of the sample 112.

The following individually describes each component of the laser microscope 100 described above. In the laser microscope 100, the laser apparatus 120 includes a laser light source and an irradiation optical system.

The laser light source radiates a plurality of pulse lasers having different wavelengths from each other. A mode-locked picosecond Nd:YVO₄ laser or a mode-locked picosecond ytterbium laser, for example, can be used as the laser light source. Furthermore, with the laser apparatus 120, it is possible to change the wavelength of the picosecond pulses using an optical parametric oscillator that sets the second harmonic of the picosecond pulse generated by the mode-locked laser to be excitation light. In this way, the laser apparatus 120 can generate a plurality of pulse lasers having different wavelengths from each other.

The irradiation optical system combines the optical paths of the plurality of pulse lasers generated by the laser light source and guides these pulse lasers on a common optical path. In this way, it is possible to form irradiation light for irradiating the sample 112 such that, among picosecond pulses having two wavelengths generated by the laser light source, the pulse with the shorter wavelength is pump light and the pulse with the longer wavelength is Stokes light, for example. In the above example, the irradiation light can be formed such that the picosecond pulses initially generated by the mode-locked picosecond Nd:YVO₄ laser are pump light and the picosecond pulses that have been wavelength-converted by the optical parametric oscillator are Stokes light.

The laser apparatus 120 can be formed using a plurality of laser light sources with different oscillation frequencies. With the laser apparatus 120, the bandwidth of the Stokes light included in the irradiation light can be increased by using photonic crystal fiber.

The confocal optical system 130 includes a pair of first objective lenses 132 arranged to sandwich the stage 110 and a second optical lens 134 arranged between one of the first objective lenses 132 and the laser apparatus 120. The first objective lenses 132 form a common focal point on the sample 112 placed on the stage 110.

The second optical lens 134 is arranged between one of the first objective lenses 132 and the laser apparatus 120, and relays the irradiation light generated by the laser apparatus 120 to the one first objective lens 132. In this way, the irradiation light generated by the laser apparatus 120 is focused near the focal point in the sample 112 and causes a non-linear effect.

A reflective mirror 192 and the second scanning system 180 are arranged in the optical path of the irradiation light between the second optical lens 134 and the laser apparatus 120. The reflective mirror 192 folds the optical path of the irradiation light to prevent the physical structure of the laser microscope 100 from becoming excessively high. The second scanning system 180 is described further below.

The first detection system 140, the second detection system, and a filter 194 are arranged on the side of the stage 110 opposite the laser apparatus 120. The filter 194 removes unnecessary components from the light emitted from the sample 112. The unnecessary components include a portion of the irradiation light emitted transparently through the sample 112, background light such as fluorescent light generated from material differing from the detection target of the first detection system 140 and the second detection system 150, and the like. Therefore, the filter 194 is changed according to the type of sample 112, the composition of the detection target, the observation method, and the like.

The first detection system 140 includes an imaging lens 142 and a polychromator 144. Furthermore, a reflective mirror 196 is arranged between the imaging lens 142 and the polychromator 144. The reflective mirror 196 folds the optical path of the emission light emitted from the sample 112 to prevent the physical structure of the laser microscope 100 from becoming excessively high.

When the broadband irradiation light has irradiated the sample 112, the polychromator 144 splits the light emitted from the sample 112 with a diffraction grating and receives the split light simultaneously with a plurality of light receiving elements. In this way, the polychromator acquires the entire spectrum of the sample 112 in the observation target region irradiated by the irradiation light.

The polychromator 144 splits and detects the light received through a narrow region corresponding to an incident slit of a beam splitter arranged at a position conjugate to the image surface of the second optical lens 134. Therefore, when the emission light is received using the polychromator, the optical path of the irradiation light irradiating the sample 112 cannot be displaced. Accordingly, the spectrum obtained by the polychromator 144 corresponds to the component at a certain position in the sample 112.

However, when the first detection system 140 is used in the laser microscope 100, by operating the stage drive section of the first scanning system 170, the sample 112 is displaced by moving the stage 110 in a direction intersecting the irradiation direction of the pulsed irradiation light, thereby realizing scanning of the sample 112 with the irradiation light through a so-called stage scan. In this way it is possible to detect Raman-scattered light or the like emitted from the predetermined observation target region in a portion of the sample 112 and create an image of the output of the first detection system 140.

The stage drive section of the first scanning system 170 can move the stage 110 in the direction of the optical axis of the irradiation light as well. In this way, when the first scanning system 170 is used, the sample 112 mounted on the stage 110 can be scanned in the optical axis direction of the irradiation light.

The reflective mirror 192 and the second scanning system 180 are arranged in the optical path of the irradiation light between the second optical lens 134 and the laser apparatus 120. The reflective mirror 192 folds the optical path of the irradiation light to prevent the physical structure of the laser microscope 100 from being excessively tall.

The second scanning system 180 displaces the optical path of the irradiation light two-dimensionally in a direction intersecting the optical axis, using the galvanic scanner 182. In this way, it is possible to scan the observation target region extracted as a portion of the sample 112 with the pulsed irradiation light including a plurality of types of excitation light, e.g. the pump light and the Stokes light, and observe the Raman-scattered light or the like released from this observation target region.

The second detection system 150 includes a imaging lens 152, a relay lens 154, an opto-electrical intensifier tube 156, and an insertable/removable reflective mirror 158. The insertable/removable reflective mirror 158 can be inserted into or removed from the optical path of the emission light from the sample 112 between the filter 194 and the first detection system 140.

When the insertable/removable reflective mirror 158 is inserted into the optical path of the emission light, the emission light emitted from the sample 112 has its optical path folded by the insertable/removable reflective mirror 158 and travels toward the imaging lens 152, the relay lens 154, and the opto-electrical intensifier tube 156 of the second detection system 150. In this case, the emission light is not incident to the first detection system 140 positioned on the downstream side of the insertable/removable reflective mirror 158.

On the other hand, when the insertable/removable reflective mirror 158 is removed from the optical path of the emission light, the emission light from the sample 112 is incident to the first detection system 140. In this case, the emission light is not incident to the second detection system 150. In this way, the first detection system 140 and the second detection system 150 are used alternatively by inserting and removing the insertable/removable reflective mirror 158.

In the second detection system 150, the light receiving surface of the opto-electrical intensifier tube 156 is arranged at a position conjugate to the pupil of the first objective lens 132. The light receiving surface of the opto-electrical intensifier tube 156 has a surface area that is significantly larger than the surface area of the light receiving surface of the polychromator 144. Accordingly, even when the galvanic scanner 182 of the second scanning system 180 operates and the optical axis of the irradiation light irradiating the sample 112 is displaced, it is possible to receive the emission light from the sample 112. In this way, the second detection system 150 can easily generate a two-dimensional image with the emission light emitted from the observation target region that is a portion of the sample 112 irradiated by the irradiation light.

The example in this drawing shows a structure in which the emission light is guided to the second detection system 150 when the insertable/removable reflective mirror 158 is inserted. However, a structure can be used in which the emission light is guided to the first detection system 140 when the insertable/removable reflective mirror 158 is inserted. However, as described above, since the surface area of the light receiving surface of the polychromator 144 used in the first detection system 140 is small, it is preferable to combine the insertable/removable reflective mirror 158 with the second detection system 150 that is easy to align relative to the emission light.

Furthermore, the laser microscope 100 shown in this drawing has transparent structures in which the irradiation of the sample 112 with the irradiation light and the detection of the emission light emitted from the sample 112 are arranged on opposite sides from each other with respect to the sample 112. However, a reflective structure can be used to radiate and detect the light on the same side of the sample 112.

In the laser microscope 100, the control system 160 includes a control section 162, a keyboard 164, a mouse 166, and a display section 168. The control section 162 can be formed by implementing a program that causes a general personal computer to perform the control procedure described further below.

The keyboard 164 and the mouse 166 are connected to the control section 162 and are manipulated when inputting user instructions to the control section 162. The display section 168 returns feedback for the manipulation by the user through the keyboard 164 and the mouse 166, and displays an image or character sequence generated by the control section 162 to the user.

The control section 162 is connected to each of the laser apparatus 120, the first detection system 140, the second detection system 150, the first scanning system 170, and the second scanning system 180, and controls operation of each of these components. Furthermore, the control section 162 generates the image to be displayed in the display section 168, based on the detection results of the first detection system 140 and the second detection system 150. The control section 162 determines the state of the sample 112 based on the detection results of the first detection system 140 and the second detection system 150.

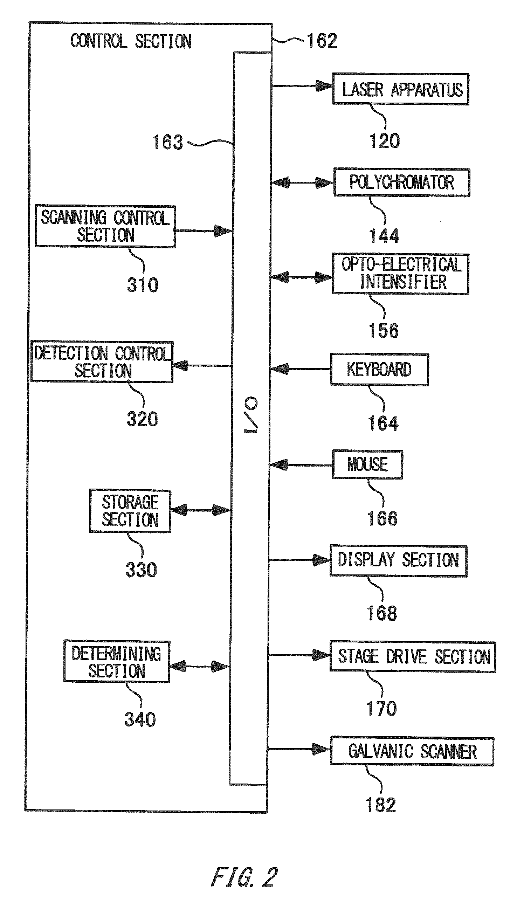

Fig. 2 is a block diagram of the control system in the laser microscope 100. In the laser microscope 100, the control section 162 includes a scanning control section 310, a detection control section 320, a determining section 340, and a storage section 330. The control section 162 is connected to the keyboard 164, the mouse 166, the display section 168, the laser apparatus 120, the stage drive section of the first scanning system 170, the galvanic scanner 182, the first detection system 140, and the second detection system 150 via the input/output section 163.

When instructed to use either the first detection system 140 or the second detection system 150, the scanning control section 310 operates the first scanning system 170 or the second scanning system 180 according to the detection system to be used. In this way, it is possible to scan the observation target region that is a portion of the sample 112 with the irradiation light and observe a predetermined observation region in the sample 112 in a single observation plane.

Accordingly, when observing a plurality of cell sheets included in one batch as the sample 112, for example, the cell sheet to be observed first is observed using the polychromator 144 in the first detection system 140 and the CARS spectrum (frequency distribution of the intensity) of the sample 112 is acquired. In this case, a spectrum with a broad frequency range is acquired using white light having a large frequency width as the light source. In this way, it is possible to extract the frequency exhibiting a peak of the desired component, e.g. lipid, in this frequency range.

Next, the second detection system 150 is used to perform measurement for the second and following cell sheets. In this case, single-color light corresponding to a frequency suitable for detection of the target molecules extracted using the first detection system 140 is used as the light source. In this way, it is possible to efficiently detect the component that is desired to be measured, and therefore it is possible to improve the detection efficiency.

The detection control section 320 references the light emission timing of the laser apparatus 120 and the scanning timing of the irradiation light, and designates a detection timing for the first detection system 140 or the second detection system 150. Furthermore, the detection control section 320 acquires the detection results of the first detection system 140 or the second detection system 150 together with the irradiation position of the irradiation light in the sample 112. In this way, the detection control section 320 maps the optical intensity of the detected light at the light emission positions in the sample 112 to generate a detection image of the observed plane.

The storage section 330 stores a control program having recorded thereon a control procedure for the control section 162 itself. The control section 162 reads the control program stored in the storage section 330 and controls the operation of the laser microscope 100. The storage section 330 stores the detection results obtained by the first detection system 140 or the second detection system 150 and allows the detection control section 320 to reference the detection results when generating the detection image.

Furthermore, the storage section 330 stores reference information that is referenced when determining whether a cell that is the sample 112 is alive or dead. The reference information may be a detection image detected from a cell that is known to be either dead or alive. The reference information may be information representing characteristic items of a detection image detected from a cell that is known to be either dead or alive. The reference information may be information indicating an evaluation value calculated by performing image processing such as a Fourier transform or differentiation on a detection image detected from a cell that is known to be either dead or alive, or indicating a method by which this evaluation value is calculated, for example.

The determining section 340 determines whether a cell is a live cell or a dead cell based on the reference information referenced from the storage section 330 and the detection image of the sample 112 generated by the detection control section 320 based on the detection results of the first detection system 140 or the second detection system 150. For example, the determining section 340 may determine whether a cell is dead or alive by acquiring from the storage section 330 a detection image obtained from a known cell, setting this detection image as the reference information, and evaluating the similarity between the reference information and the detection image acquired from the sample 112.

As another example, the determining section 340 may determine whether a cell is dead or alive by acquiring from the storage section 330 characteristic items extracted from a detection image obtained from a known cell, setting these characteristic items as the reference information, and checking if this reference information matches characteristics of the detection image acquired from the sample 112. As yet another example, the determining section 340 may determine whether a cell is dead or alive according to a predetermined threshold value obtained by comparing an evaluation value calculated by performing image processing such as a Fourier transform or differentiation on a detection image detected from the sample 112 to an evaluation value obtained from the storage section 330.

The determining section 340 may have the user perform some or all of the determination process, by displaying the detection image and the reference information to the user through the display section 168. When the determination is left up to the user, it is possible to use the detection image obtained from a known cell as the reference information.

The determination result of the determining section 340 is output to the outside through the display section 168. The output to the outside may be the number of live cells or dead cells obtained by counting the determination results or a live percentage or dead percentage of the cells calculated from the ratio between the determined number of live cells and the determined number of dead cells. Furthermore, the sample 112 that is the observation target may be evaluated according to the determination results, and the information indicating whether a quality standard is satisfied may be output using, as a threshold for the quality, a predetermined number of dead cells, the ratio of dead cells to live cells, i.e. the percentage of dead cells, the percentage of live cells, or the like.

With the laser microscope 100 described above, the detection light that is detected from the sample 112 irradiated with the irradiation light by the first detection system 140 or the second detection system 150 can be exemplified by coherent anti-Stokes Raman-scattered light (referred to hereinafter as CARS light).

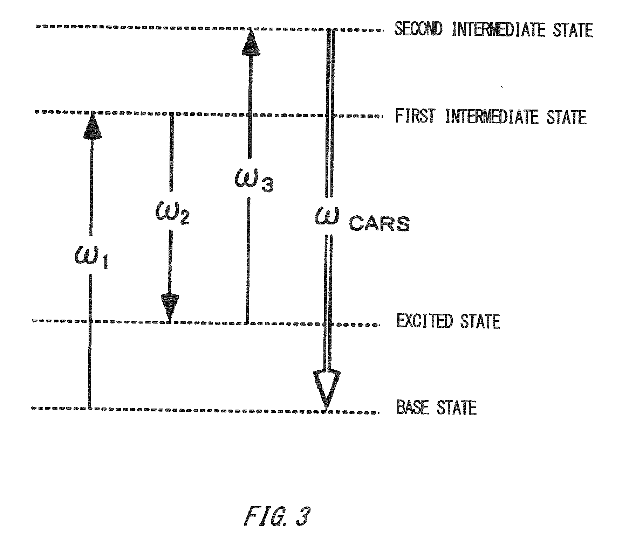

Fig. 3 is a schematic view for describing a CARS process for generating the CARS light by the sample irradiated by the irradiation light. The CARS process occurs when the sample is irradiated with irradiation light that includes two types of laser light, which are the pump light and the Stokes light, having respective optical frequencies ω₁ and ω₂ that are different from each other and the difference (ω₁-ω₂) between the optical frequency ω₁ of the pump light and the optical frequency ω₂ of the Stokes light matches an angular frequency ω₀ of the normal mode oscillation of the molecules included in the sample.

As a result of the CARS process, when the oscillation mode of a prescribed molecular structure included in the sample is excited, the molecular oscillation interacts with the probe light, which is third laser light having an optical frequency of ω₃, thereby causing the CARS light derived from a third-order non-linear pole to be generated as Raman-scattered light.

Furthermore, since the pump light is also used as the probe light, the CARS light is generated under the condition that ω₁ = ω₃. The CARS light generated by the sample has an optical frequency that satisfies the expression ω_{CARS} = ω₁-ω₂+ω₃. Accordingly, by detecting the CARS light emitted from the sample, it is possible to detect the presence of a certain molecular structure, e.g. a functional group, included in the sample. Furthermore, by repeatedly detecting the CARS light while changing the position in the sample irradiated by the irradiation light, it is possible to form an image of the distribution of the certain molecular structures in the sample 112.

The CARS light has higher optical intensity than naturally emitted Raman-scattered light and the like, and therefore it is possible to detect the light more quickly than in a case where an optoelectrical converting element is used. Accordingly, not only is the time needed for the detection shortened, but it is also possible to perform observation at the video rate. Therefore, it is possible to detect not only the distribution of a certain molecular structure, but also the change in the distribution. Furthermore, by setting the band of the irradiation light irradiating the sample to be the infrared band that does little damage to live cells, it is possible to observe live cells that are observation targets in their living state.

Furthermore, the CARS light caused by the non-linear effect is generated in a very narrow region constricted by the irradiation light due to the objective lenses. Therefore, the observation target region, which is the target of the CARS light detection, is a region that is narrow in both the direction intersecting the optical axis of the irradiation light and the direction parallel to the optical axis. Accordingly, the observation of the sample using the CARS light has stereoscopically high resolution.

Therefore, the observation plane may be formed within the sample using the irradiation light in the infrared band or near the infrared band. Furthermore, by sequentially forming observation planes in the depth direction of the sample, it is possible to generate an image that reflects the three-dimensional distribution of the certain molecular structure.

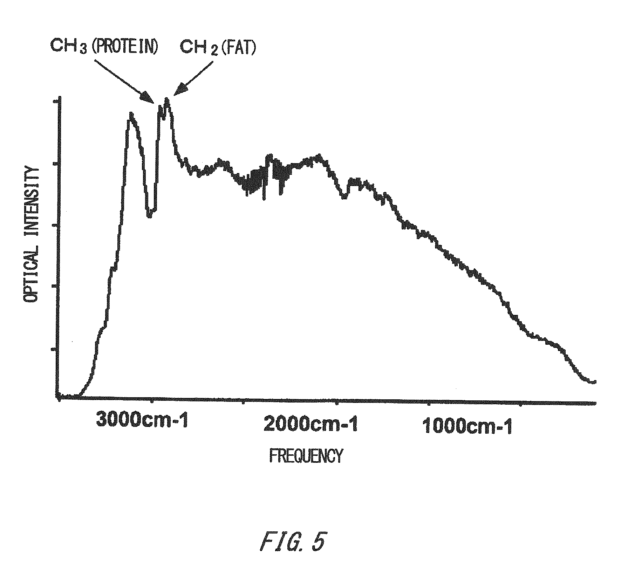

In addition, by changing the optical frequency of the irradiation light irradiating the sample at a certain position, it is possible to obtain a spectrum image showing the frequency distribution of the Raman-scattered light emitted from this irradiation position, e.g. the frequency distribution shown in Fig. 5. Furthermore, by repeatedly performing irradiation with the irradiation light while moving the sample in a direction intersecting the optical path of the irradiation light, it is possible to generate a detection image by creating an image of the distribution of certain molecules in the observation plane that includes the focal point of the first objective lens 132.

It is possible to determine the state of a sample based on the characteristics of the detection image generated in the manner described above. The following describes the determination concerning whether a cell included in the sample is dead or alive, using the laser microscope 100.

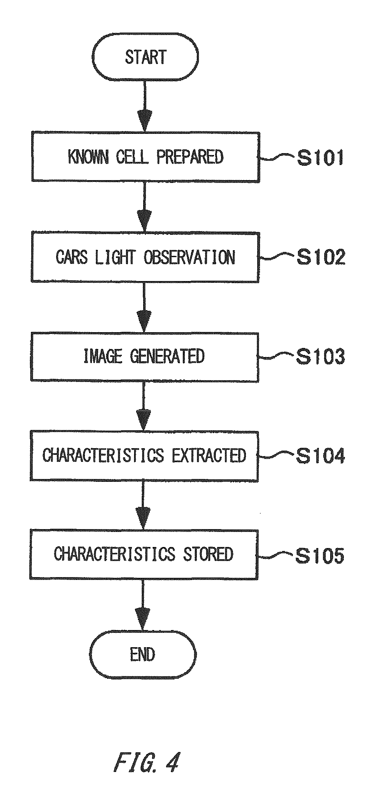

Fig. 4 shows an exemplary flow of a preparation process performed before the observation of the sample 112 using the laser microscope 100. When determining whether a cell included in the sample 112 is alive or dead through observation of the sample 112 using the laser microscope 100, first, a reference sample is prepared that is known to include live cells or dead cells (step S101). The reference sample may be realized by preparing a single sample that includes a live cell or by preparing a plurality of samples that each include a live cell, for example. Furthermore, both a sample that includes a live cell and a sample that includes a dead cell may be prepared.

Next, the reference sample is observed using the CARS light (step S102), and the detection image is generated (step S103). If there are a plurality of reference samples, each sample is observed using the CARS light and a detection image is generated individually for each reference sample. In this way, it is possible to obtain a detection image including inherent characteristics of at least one of a live cell and a dead cell.

The detection image generated from the reference sample may be stored in the storage section 330 as-is as the reference information. Characteristics may be extracted using image processing from each detection image generated by the detection control section 320 (step S104). The types of the extracted characteristics and the evaluation values thereof may also be stored in the storage section 330 as reference information. In this way, the storage section 330 realizes a state in which the reference information can be referenced from the determining section 340 or the like.

The reference information can be generated by performing CARS light observation of a cell that is known to be dead or alive as a result of being dyed and observed with a fluorescent microscope, to acquire a CARS image of at least one of a live cell and a dead cell. Furthermore, a reference image may be generated through CARS light observation by performing CARS light observation of a sample 112 after checking the state of a dead cell, e.g. whether the cell died of apoptosis or necrosis, in a sample 112 for generating reference information using a reagent that makes the characteristics of dead cells easily seen.

In addition to the observation image, the spectrum and the like may be extracted from the sample 112 used to generate the reference information and may be stored as reference information. The reference information may be generated according to the observation target region corresponding to the field of view of the observation method, or may be generated in cell units.

The reference information obtained in this manner may be used in a database generated in advance and shared among various observations. Furthermore, reference information may be generated for each batch from a sample 112 for generating reference information extracted from a sample 112 group shared by the batch.

Fig. 5 shows a graph obtained by plotting the spectrum of a sample based on the CARS light detected from the sample using the laser microscope 100. In the graph shown in this drawing, there is a peak caused by the presence of CH₂ bonds that is a characteristic associated with the presence of lipid in the sample and a peak caused by the presence of CH₃ bonds that is a characteristic associated with the presence of protein. In this way, it is possible to detect certain molecular structures included in the sample 112 through observation with the CARS light.

The graph shown in the drawing may also be saved in the storage section 330 as one piece of reference information. In this way, when a spectrum image is detected to have the same peaks by observing another sample with the laser microscope 100, the determining section 340 can determine that this sample is a cell having the same molecular structure as the reference sample. Furthermore, when a molecular structure unique to live cells or dead cells is determined, the determining section 340 can determine whether a cell is dead or alive based on the spectrum image.

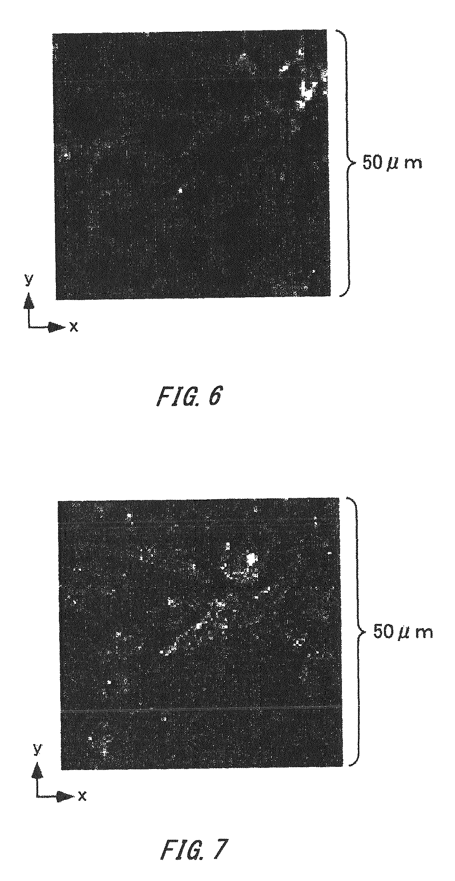

Fig. 6 shows a detection image generated from another sample 112 by the detection control section 320 of the laser microscope 100. The detection image in this drawing was generated as a single observation plane in a cell sheet formed by live cells.

The laser apparatus 120 of the laser microscope 100 generated the irradiation light including pump light with a wavelength of 1064 nm and Stokes light with a wavelength of 1550 nm. In this way, CARS light with a wavelength of 820 nm was generated by the CH bonds included in the lipid in each of the cells included in the sample 112.

In the CARS light observation described above, the galvanic scanner 182 was moved and the sample 112 was repeatedly irradiated with the irradiation light while the optical path thereof was being displaced. The region that generated the CARS light in the sample 112 was a square-shaped region with a side length of 50 µm in a single observation plane orthogonal to the optical axis of the irradiation light within the sample 112.

The CARS light generated from the sample 112 was detected by the opto-electrical intensifier tube 156 of the second detection system 150. The detection control section 320 generated the detection image by plotting the optical intensity of the received CARS light with the galvanic scanner 182 according to the scanning by the second scanning system 180. In this way, with the laser microscope 100, a detection image was generated that shows a two-dimensional distribution of the Raman-scattered light in one observation plane of the sample 112.

The detection image generated by the detection control section 320 includes bright points that reflect the distribution of lipid included in the cell sheet. Furthermore, the black spots appearing in the detection image correspond to the arrangement of cell nuclei that do not contain lipid, i.e. live cells. Accordingly, by counting these black spot images, it is possible to count the number of live cells included in the observation region. In this way, the characteristics of the distribution of the cell nuclei and lipid, which is the primary component in the cytoplasm, in the live cells clearly appears.

When a Fourier transform is performed on the detection image detected from live cells such as described above, the image is characterized by having few high spatial frequency components. Accordingly, a threshold value is set in advance and cells in the sample are determined to be live cells when the spatial frequency of the detection image detected from an unknown sample is less than this threshold value.

Fig. 7 shows another detection image detected from a cell sheet observed when detecting the detection image shown in Fig. 6. This detection image was generated with CARS light resulting from irradiation light including pump light with a wavelength of 1064 nm and Stokes light with a wavelength of 1550 nm. In this way, by generating the CARS light at the CH₃ bonds included in the protein of the cell, the detection image in the drawing reflects the distribution of protein in the same region as shown in Fig. 6 in the cell sheet serving as the sample 112.

Lipid and protein are present in an exclusive manner in a single cell sheet, and therefore the detection image shown in Fig. 7 has a distribution of bright points that is substantially the inverse of the bright point distribution of the detection image shown in Fig. 6. Accordingly, by calculating the difference between the detection image shown in Fig. 6 and the detection image shown in Fig. 7, it is possible to suppress the noise in the detection images. In this way, by generating a plurality of detection images with CARS light for a single sample 112, it is possible to improve the detection accuracy of the laser microscope.

As already described above, the irradiation light that causes the CARS light to be generated is realized by using a pulse laser in the infrared band. Therefore, the live cells that are observation targets are not damaged by the irradiation light. Accordingly, even when the laser microscope is used in order to investigate the content ratio of dead cells among reproduced cells before use, the ratio of live cells is not changed by the observation with the CARS light generated by the irradiation with the irradiation light in the infrared band.

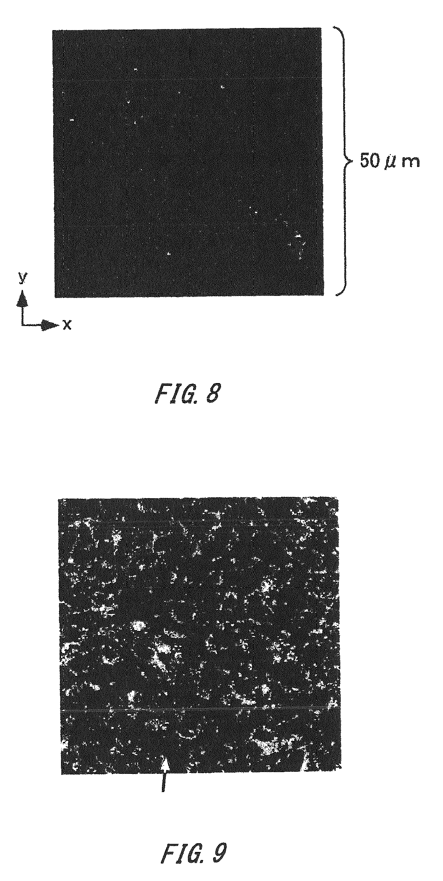

Fig. 8 shows a detection image generated from another sample 112 by the detection control section 320 of the laser microscope 100. The detection image of this drawing was generated using a single-layer cell sheet formed from dead cells that had died from necrosis as the sample 112.

The observation conditions of the laser microscope 100 were the same as the in the case of the observation of the cell sheet of live cells shown in Fig. 6, including the wavelengths of the irradiation light. Accordingly, the detection image shown in Fig. 8 reflects the distribution of lipid in the dead cells.

From the detection image shown in this drawing, characteristics are seen of a distribution in which the presence of lipid causes scattered bright spots the are grouped in a localized manner. In this detection image, the bright spots are extremely bright compared to the surrounding regions, and therefore the overall brightness of the detection image was reduced to clearly show the peak of each bright point.

When a Fourier transform is performed on the detection image detected from the dead cells such as described above, the result is characterized by including high spatial frequency components. Accordingly, a threshold value is set in advance and cells in the sample are determined to be dead cells when the spatial frequency of the detection image detected from an unknown sample is greater than this threshold value.

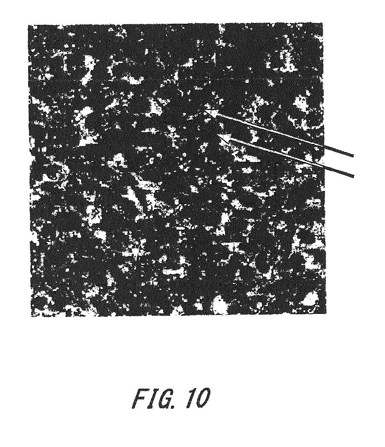

Figs. 9 and 10 show detection images generated from another sample 112 by the detection control section 320 of the laser microscope 100. The detection images of these drawings were generated using a single-layer cell sheet formed from dead cells that had died from necrosis as the sample 112.

From the detection images in these drawings, a characteristic was found that lipid droplets entered into the cytoplasm and into the cell nucleus in each of the dead cells shown by the arrows in the drawings. Accordingly, when an image is observed that shows that lipid droplets have entered into the cytoplasm or the cell nucleus in an observation image of another sample 112, it can be determined that the cells included in this sample 112 are dead cells.

At this time, the cells may be determined to be dead cells if the ratio of lipid droplets making up the inside of the cytoplasm or the cytoplasm or the cell nucleus or the amount of lipid droplets that have entered into the cytoplasm or the cell nucleus is greater than a prescribed threshold value. If the amount or ratio of lipid droplets is not greater than the prescribed value, the cell is not determined to be a dead cell and another investigation for determining whether the cell is dead or alive is made after treating the cell by providing further nutrients, for example.

When the sample 112 of a cell sheet is observed using the CARS light, both the cell nuclei and the empty spaces within the cells appear black in the image detecting the lipid distribution. Accordingly, by identifying the nuclei and the empty spaces, it is possible to improve the accuracy of the determining concerning whether a cell is living or dead and the number of cells.

Therefore, when using the laser microscope 100, it is possible to identify the empty spaces and the cell nuclei in the lipid distribution image by acquiring an image having four wave mixing that enables observation of the existence or non-existence and the shape of cells by detecting a refractive index distribution and comparing this image having four wave mixing to the lipid distribution image. In the same manner, the empty spaces and the cells can be identified by acquiring an image having four wave mixing and a protein distribution image in the sample 112 and comparing these images.

Since the protein distribution image is an inversion of the lipid distribution image and is an image in which the cell nuclei are shown brightly, it is possible to increase the contrast of the lipid distribution image or the protein distribution image by obtaining the difference between the protein distribution image and the lipid distribution image. In this way, it is possible to identify the cell nuclei and the empty spaces.

In this way, when observing a cell sheet using the laser microscope 100, a detection image obtained from live cells and a detection image obtained from dead cells respectively have significantly different characteristics. Accordingly, when a cell that is not known to be dead or alive is observed as the sample, it is possible to determine whether the cell is dead or alive by searching the reference information for an image having characteristics that match or resemble the characteristics of the detection image obtained from the sample.

In the example described above, the sample is irradiated with irradiation light that causes Raman-scattered light to be generated by the molecules included in lipid and protein. However, other types of molecules that can be made to generate Raman-scattered light may be selected as detection targets, according to the types of cells included in the sample. Furthermore, in the examples in the drawings, the detection images are generated by irradiating the sample 112 with a single irradiation light. However, the sample may be irradiated with a plurality of irradiation lights to generate a plurality of detection images in parallel. In this way, a large sample can be observed quickly.

The indicators for identifying the state of a cell based on the detection image are obviously not limited to the above example. For example, it is possible to determine whether a cell is dead or alive based on characteristics that a live cell moves and a dead cell does not move, as one example of an indicator for determining whether a cell is alive or dead.

In this case, it is possible to observe the same observation region in a cell serving as the sample two or more times at certain time intervals and determine that the cell is a dead cell if there is no change found when comparing the images obtained from these observations. When using this type of determination indicator based on the movement of a cell, it is possible to easily identify movement of the cell based on the images obtained by detecting the distribution of lipid that is contained in large amounts in the organelles of the cell by focusing particularly on whether the position of the cell organelles, such as the mitochondria, change positions.

Furthermore, as another example of a determination indicator for determining whether a cell is alive or dead, the cell may be determined to be alive or dead based on the distribution of bright points having a granular shape detected from the detection image. As already described above, it is possible to detect the presence of lipid as bright regions in an image obtained through detection of CARS light.

In a cell that has died from apoptosis, the organelles including a large amount of lipid are covered in a lipid layer and become granular. In contrast to this, in a cell that has died from necrosis, mitochondria that primarily include lipid are swollen and granular. Therefore, in an image generated by detecting the lipid distribution, the live cells have few granular bright points, while the number of granular bright points is clearly higher in dying cells or dead cells resulting from apoptosis or necrosis. Therefore, it is possible to determine whether a cell is dead or alive based on the amount of granular bright points in an image obtained by observing the lipid distribution. When observing the cell state using this, it is possible to determine whether a cell is dead or alive.

In the examples described above, the cells are mostly determined to be dead or alive based on the detection images generated using the laser microscope 100. However, the items that can be determined for a cell using the laser microscope 100 are not limited to whether the cell is simply dead or alive. For example, even for the same dead cells, depending on the characteristics of the detection image, it is possible to determine whether the dead cells died from apoptosis or from necrosis.

For example, the outline of a cell that has died of apoptosis is closer to a circular shape compared to a live cell, which is flat and elongated when viewed two-dimensionally. Furthermore, pyknosis occurs within such cells. In addition, the cell membrane contracts to be smaller than that of a live cell. Yet further, in a cell that has died of apoptosis, several of the organelles within the cytoplasm are enclosed in a lipid layer, the cell separates from other surrounding cells, and finally breaks up into a plurality of apoptotic endoplasmic reticula.

In a cell that has died of necrosis, the mitochondria are swollen, the cell membrane ultimately tears, and the internal material including DNA spills out into the surrounding region. Accordingly, when observing a cell state using an image obtained by observing a lipid distribution, in addition to determining whether a cell is alive or dead, it is also possible to determine whether a dead cell died of apoptosis or of necrosis.

In addition, apoptotic death occurs sporadically within a cellular composition in which a plurality of cells are gathered. Accordingly, the surrounding cells that are adjacent to a cell that has died from apoptosis are often alive. On the other hand, cells that have died of necrosis affect other nearby cells, and therefore form groups including a plurality of dead cells. Accordingly, it is possible to determine whether dead cells died of apoptosis or of necrosis based on an image showing the distribution state of dead cells.

When a dead cell has died of necrosis, there is a possibility that the death of the cell was caused by an outside cause such as mixing with a pathogen such as a virus, for example. Accordingly, when a dead cell is determined to have died from necrosis, an investigation of the manufacturing process of the cell sheet including the culturing process of the cell is performed.

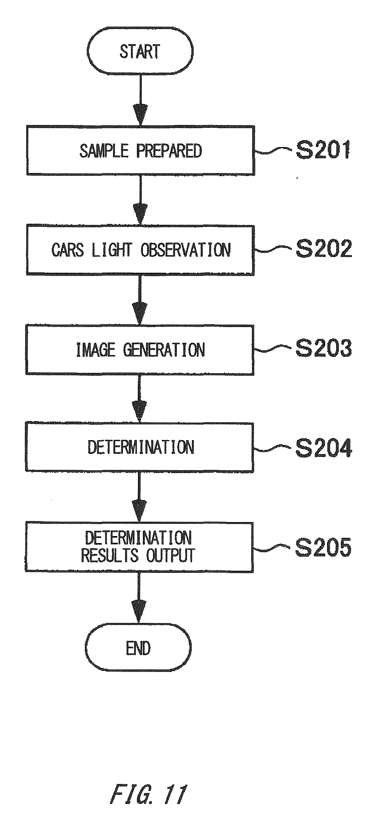

Fig. 11 shows an exemplary flow of the observation process using the laser microscope 100. When making a determination about a cell using the laser microscope 100, first, the sample 112 is prepared (step S201). The sample 112 may be housed in a culture vessel along with a culture solution, in order to maintain the living environment of the live cells. The prepared sample 112 is mounted on the stage 110 of the laser microscope 100.

Next, the sample 112 is irradiated with the irradiation light and detection light generated by the sample 112 is detected with the first detection system 140 or the second detection system 150 (step S202). Next, the detection image is generated based on the distribution of the detected detection light (S203).

After this, the determining section 340 references the reference information and makes a determination concerning the detection image (step S204). As already described above, there are several determination methods used by the determining section 340. The determining section 340 need only perform one of these determination methods. Furthermore, if a determination cannot be made by one of these methods, another method may be tried.

The determination results of the determining section 340 may be displayed in the display section 168 by the control section 162. The content displayed in the display section 168 may be characters describing the determination results, or may be values obtained by calculating the living percentage of the cells if the sample 112 includes a plurality of cells. If a predetermined threshold value is set and the number of dead cells included in the sample 112 or the ratio of dead cells to live cells, i.e. the dead percentage, exceeds this threshold value, a warning or the like indicating this fact may be displayed. Furthermore, if it is assumed that the cells of the sample 112 are to be used as a cell sheet (medical product), a determination result indicating whether the sample 112 has high enough quality to be used may be displayed.

If the sample 112 is a cell sheet, the cell sheet may be shipped as a medical product if it is determined to be good quality, but if the cell sheet is determined to be poor quality, the cell sheet may be discarded or the dead cells among the cells forming the cell sheet may be removed and the resulting cell sheet shipped as a product. If the dead cells are to be removed, another investigation may be performed concerning whether the number or ratio of dead cells is less than or equal to a prescribed threshold value. If a determination is made concerning whether a dead cell is a cell that will negatively affect surrounding live cells or whether the dead cell contains a pathogen, for example, when it is determined that the dead cell will not have a negative effect, this cell sheet may be determined to be usable as a product even if the number or ratio of dead cells is greater than or equal to the threshold value.

Furthermore, the detection image obtained from the sample 112 may be displayed together with a reference detection image that the determining section 340 has determined matches or resembles the detection image from the sample 112, and the final decision may be made by the user. In this case, the region determined by the determining section 340 to be an image of live cells or dead cells may be shown in an emphasized manner by having a color or mark attached thereto.

When the determination results are confirmed in this way, the control section 162 outputs the determination results to the outside (step S205). In this way, one series of control of the observation by the laser microscope 100 is finished. The observation method described above can be provided as a program that causes an electronic calculator to perform the method, via a magnetic recording medium, an optical recording medium, a communication line, or the like.

In the detection image shown before in Fig. 8, many granular bright points are seen spanning a plurality of cells. Accordingly, in the detection image obtained by observing an unknown sample 112, when many granular bright spots are seen, it is possible to determine that these cells are dead cells caused by necrosis. An operation such as counting the number of bright points for each detection image has low efficiency, and is therefore unsuitable as a method to use when making determinations for many samples 112. However, it is possible to automate the determination by performing image processing on the detection images.

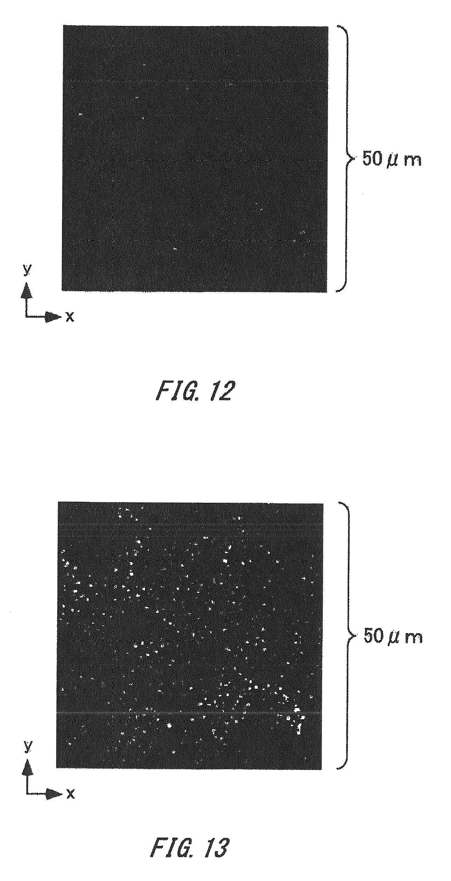

Figs. 12 to 16 show each step in a process for applying image processing to the detection image shown in Fig. 8. Fig. 12 shows a processed image resulting from processing the detection image shown in Fig. 8 with a band-pass filter using an FFT (Fast Fourier Transform). The band-pass filter was set to have a structure somewhat larger than the granular bright points seen in Fig. 8 as a threshold, in order to extract the highfrequency component. In this way, the granular bright points included in the detection image are extracted.

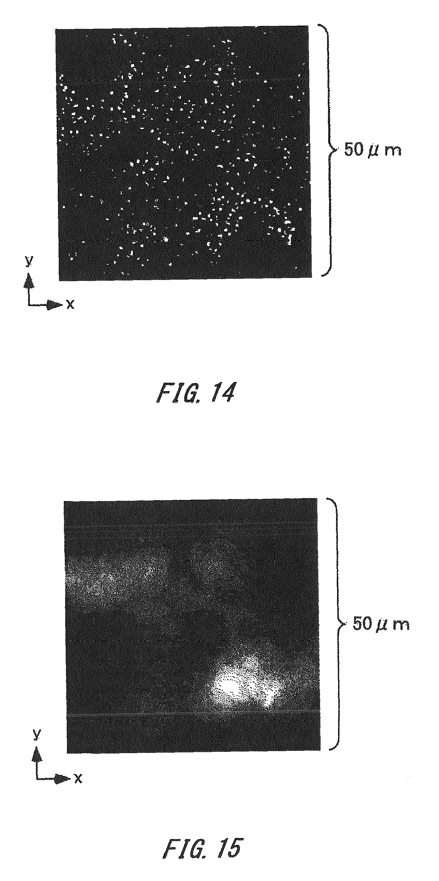

Fig. 13 shows a processed image in which the extracted granular bright points are easier to see, obtained by subtracting a brightness of approximately 60% of the average brightness of all pixels from all of the pixels in the processed image shown in Fig. 12. Fig. 14 shows a processed image resulting from the processed image shown in Fig. 13 being made binary using a suitable threshold value. As shown in this image, only the granular bright point objects in the original detection image are extracted. Fig. 15 is an image obtained by processing the processed image shown in Fig. 14 with an averaging filter that performs averaging for circular regions having a size of approximately one cell. Furthermore, Fig. 15 shows the result obtained by performing a brightness adjustment to make the contrast clear.

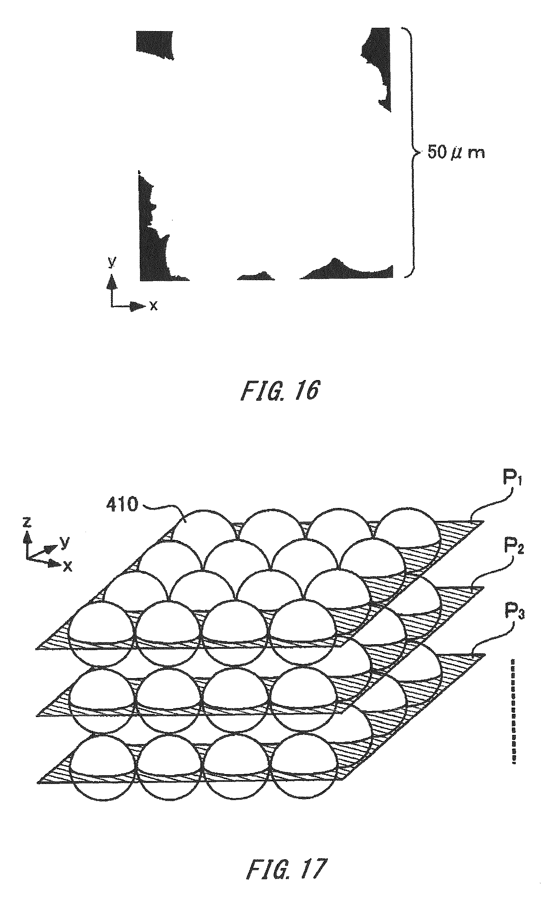

Fig. 16 shows a processed image resulting from the processed image shown in Fig. 15 being made binary using a suitable threshold value. The bright regions in the processed image shown here correspond to regions where the granular bright points are distributed in the detection image. In this example, it is seen that the area of the regions where the granular bright points are distributed is larger than the size of a single cell. Accordingly, it can be determined that this cell is a dead cell that died of necrosis.

If the sample that is the detection target is a dead cell that died of apoptosis, the area of the regions where the granular bright points are distributed that is extracted at the stage shown in Fig. 16 is approximately the size of one cell. Furthermore, if the sample that is the detection target is a live cell, the area of the regions where the granular bright points are distributed that is extracted at the stage shown in Fig. 16 is smaller than one cell or nonexistent.

The image processing described above can be applied to the detection images detected from other samples 112 by repeatedly using the initially set conditions. Accordingly, it is possible to automate the processing when making determinations for a large number of samples 112.

Fig. 17 is a schematic view for describing another example of observing a cell using the laser microscope 100. With the laser microscope 100, the region in which the CARS process occurs is stereoscopically very narrow. Accordingly, the range in which the CARS process occurs in the sample 112 is extremely narrow in the direction of the optical path of the irradiation light.

In a cellular composition serving as the sample 112 of the laser microscope 100, cells 410 are present three-dimensionally in adjacent and layered manners. On the other hand, the range of the region from which the detection image is formed by the detection light is smaller than an individual cell 410, as shown by the observation planes P₁, P₂, P₃, etc. in the drawing. Accordingly, it is possible to form a detection image that reflects the stereoscopic structure within the cell by, after detecting the one observation plane P₁, individually detecting each observation plane P₂ and P₃ by sequentially changing the position of the observation plane.

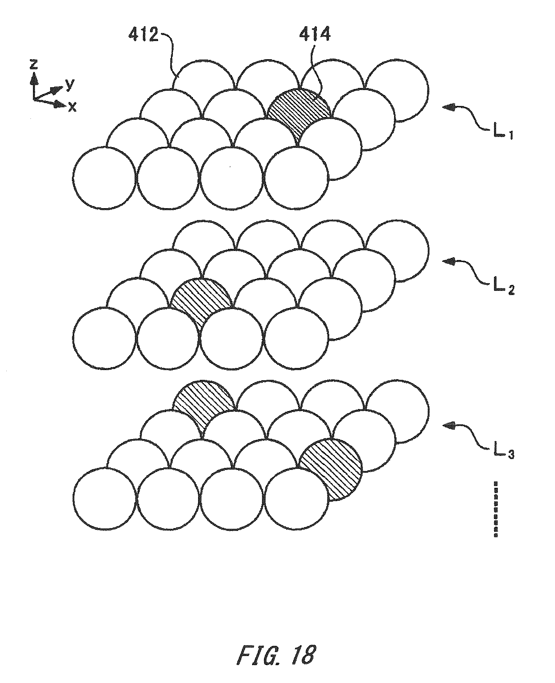

Fig. 18 is a schematic view showing the basics of the stereoscopic detection described above. As shown in the drawing, by detecting the live cells 412 and the dead cells 414 distributed among the live cells in each layer, thereby detecting the live percentage of cells in the stereoscopic cellular composition for each layer L₁, L₂, L₃, etc., it is possible to accurately detect the live percentage of the cellular composition.

It is obvious that cellular compositions are not arranged as orderly as shown in the drawing. However, by decreasing the movement amount of the observation planes P₁, P₂, P₃, etc. in the Z direction, it is possible to reproduce an image of the stereoscopic structure of the cellular composition. In this way, with the laser microscope 100, a detection image is generated that shows a three-dimensional distribution of the Raman-scattered light in a plurality of observation planes in the sample 112.

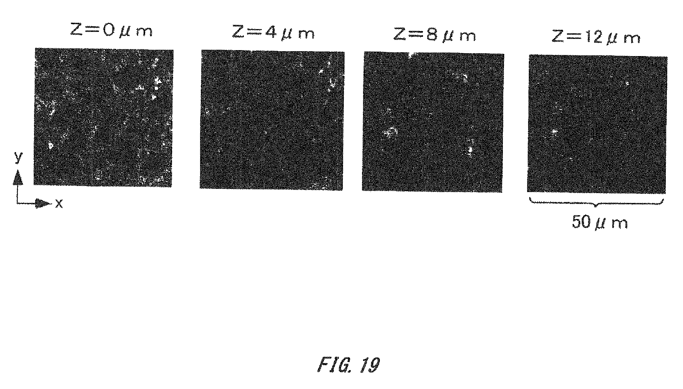

Fig. 19 shows a multilayer detection image detected from a multilayer cell sheet formed of live cells. The irradiation light used has the same conditions as the irradiation light used for the detection of the detection image shown in Fig. 6. Furthermore, the galvanic scanner 182 was used to generate a detection image from a square region in which a side has a length of 50 µm in each observation plane. Each observation plane in which the CARS process occurs is formed parallel to the x-y plane orthogonal to the optical path of the irradiation light.

The detection image was detected from the surface of the sample 112 and three observation planes set at intervals of 4 µm from the surface. From these four detection images, the arrangement of the nuclei and the cellular characteristic that lipid is distributed uniformly are found in each observation plane of the sample 112. Accordingly, for this sample 112, it is seen that each layer is formed of live cells.

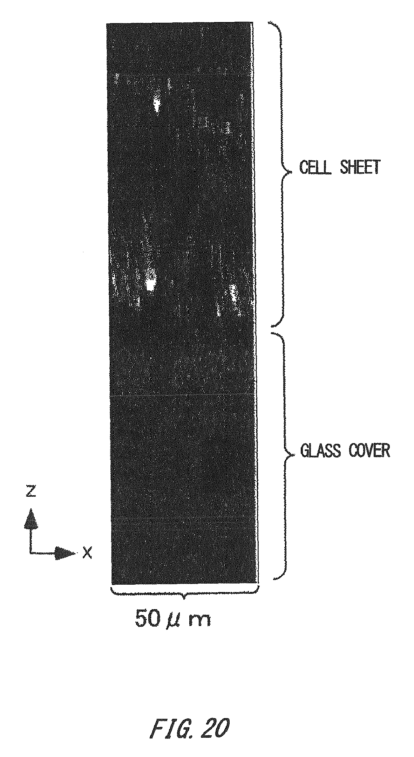

Fig. 20 shows a detection image detected from the sample 112 described above using a different method. The irradiation light here is the same as the irradiation light used for the detection described above. However, the galvanic scanner 182 was pivoted on one axis such that the sample 112 was moved only in the x direction. The sample stage 110 was moved parallel to the optical axis of the irradiation light while the detection was being performed, and the detection light was detected while moving the region in which the CARS process occurs in the z direction within the sample 112.

The detection image generated from the detection light generated through the method described above reflects the structure of a region with a width of 50 µm parallel to the x-z plane in the sample 112. The uniform structure at the bottom portion of this image corresponds to the cover glass on which the sample 112 was mounted.

In this way, the laser microscope 100 can generate a detection image that reflects the structure of the cellular composition in a direction parallel to the optical axis of the irradiation light. Furthermore, by changing the scanning direction of the irradiation light, it is possible to generate a detection image that reflects the molecular structure in an observational plane tilted at an arbitrary angle within the sample 112.

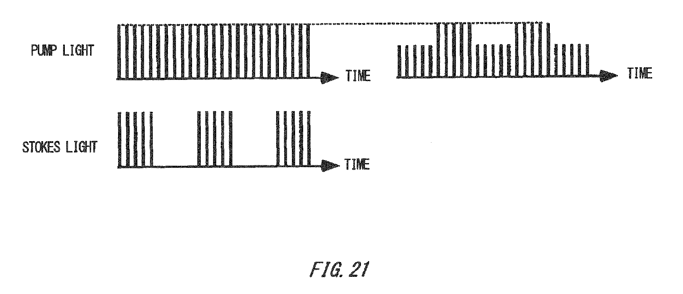

Fig. 21 is a diagram for describing a method for generating detection light through a process different from the CARS process, using the laser microscope 100. The following describes a method that includes generating detection light from the sample 112 by using stimulated Raman-scattered light.

When the interference component generated by the pump light and the Stokes light included in the irradiation light of the laser microscope 100 match the molecular oscillation of the physical material, resonance occurs and causes stimulated Raman scattering that is amplified by the Stokes light. When measuring the Raman-scattered light, it is possible to detect an increase in the Stokes light that accompanies a decrease in the pump light. By detecting the amount of change in the intensity of the pump light or the Stokes light, it is possible to generate a detection image by detecting the detection light reflecting the molecular structure of the sample 112, in the same manner as the detection of the CARS light.

When detecting the detection light caused by stimulated Raman scattering, the intensity of the Stokes light that is a portion of the irradiation light is modulated. A chopper, acousto-optic element, or the like can be used as the modulator. The Stokes light whose intensity has been modulated combines with the pump light to irradiate the sample 112 as the irradiation light.

At the focal point of the first objective lens 132, stimulated Raman scattering occurs due to the interference between the Stokes light described above and the pump light. When resonance occurs with the molecules in the sample 112, if the intensity of the Stokes light increases, the optical intensity of the pump light decreases. Accordingly, when the intensity of the Stokes light is modulated, the intensity of the pump light changes according to the change in the intensity of the Stokes light.

The change in optical intensity of the pump light is detected by the opto-electrical intensifier tube in the second detection system 150, for example. However, the amount of change in the pump light caused by the stimulated Raman scattering is small, and therefore a lock-in amplifier may be used that can detect a very small signal. In this way, the optical intensity detected by the second detection system 150 reflects the molecular structure of the sample 112 at the focal point of the first objective lens 132. Accordingly, by scanning the sample using the first scanning system 170 or the second scanning system 180, it is possible to generate a detection image that reflects the molecular structure of the sample 112, in the same manner as when using CARS light.

In the case described above, the intensity of the Stokes light is modulated and the sample 112 is observed using the stimulated Raman loss obtained by performing a lock-in detection on the pump light. However, it is also possible to observe the sample 112 in a similar manner by modulating the intensity of the pump light and used the stimulated Raman gain obtained by performing a lock-in detection on the Stokes light.

In addition, the cell observation method of the present invention can also be used when determining whether a cell is dead or alive with a method other than the method for detecting Raman-scattered light. For example, it is possible to detect the distribution of lipid or protein in a cell according to the absorption spectrum in a sample 112, using an infrared microscopy technique that includes irradiating the sample 112 serving as the observation target with infrared rays and detecting the light reflected or scattered by the sample 112. In this way, it is possible to determine whether a cell included in the observation target is alive or dead and to also detect the number of dead cells or live cells within the sample 112, the state of lipid or protein in the sample 112, and the like.

Furthermore, it is possible to form a manufacturing apparatus for manufacturing cell sheets by using the cell observation method described above. Specifically, by including a preparing section for separating cells to prepare a cell line, a culturing section to culture the cell line into a cell sheet, and a determining section that determines whether cells in the cell sheet are dead or alive using the cell observation method described above, it is possible to efficiently manufacture a high-quality cell sheet that includes many live cells or few dead cells. In this manufacturing apparatus, the cell observation method described above may be adopted to observe cells during the culture or during another manufacturing stage.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

### [List of Reference Numerals]

100: laser microscope, 110: stage. 112: sample, 120: laser apparatus, 130: confocal optical system, 132: first objective lens, 134: second optical lens, 140: first detection system, 142, 152: imaging lens, 144: polychromator, 150: second detection system, 154: relay lens, 156: opto-electrical intensifier tube, 158: insertable/removable reflective mirror, 160: control system, 162: control section, 163: input/output section. 164: keyboard, 166: mouse, 168: display section, 170: first scanning system, 180: second scanning system, 182: galvanic scanner, 184: scanner lens, 186: primary image surface, 192, 196: reflective mirror, 194: filter, 310: scanning control section, 320: detection control section, 330: storage section, 340: determining section, 410: cell, 412: live cell, 414: dead cell

## Claims

1. A cell observation method comprising:
a detection step of detecting radiation light that is radiated from an observation target when the observation target is irradiated with irradiation light; and
a determination step of determining whether a cell included in the observation target is alive or dead based on the radiation light.

2. A cell observation method comprising:
a detection step of detecting radiation light that is radiated from an observation target when the observation target is irradiated with irradiation light; and
a detection step of detecting the number of live cells included in the observation target based on the radiation light.

3. A cell observation method comprising:
an acquisition step of acquiring a spectrum of irradiation light that irradiates an observation target when the observation target is irradiated with the irradiation light; and
a detection step of detecting a state of at least one of lipid and protein included in the observation target based on the spectrum.

4. The cell observation method according to Claim 1, wherein
the detection step includes detecting Raman-scattered light radiated from the observation target when the observation target is irradiated with first excitation light having a first wavelength and second excitation light having a second wavelength that has a wavelength difference relative to the wavelength of the first excitation light corresponding to normal mode oscillation of molecules included in a cell of the observation target, and
the determination step includes determining whether a cell included in the observation target is dead or alive based on the Raman-scattered light detected in the detection step.

5. The cell observation method according to Claim 4, wherein
the Raman-scattered light includes at least one of coherent anti-Stokes Raman-scattered light and stimulated Raman-scattered light.

6. The cell observation method according to Claim 4 or 5, wherein
the determination step includes comparing the detection result of the detection step to at least one of a detection result of Raman-scattered light generated from a cell determined in advance to be a live cell and a detection result of Raman-scattered light generated from a cell determined in advance to be a dead cell, to determine whether a cell of the observation target is dead or alive.

7. The cell observation method according to any one of Claims 4 to 6, wherein
the detection step includes an image generation step of generating a detection image showing a frequency distribution of the Raman-scattered light in the observation target.

8. The cell observation method according to any one of Claims 4 to 7, wherein
the detection step includes a step of detecting a two-dimensional distribution of Raman-scattered light in one observation plane in the observation target.

9. The cell observation method according to any one of Claims 4 to 8, wherein
the detection step includes a step of detecting a three-dimensional distribution of Raman-scattered light in a plurality of observation planes in the observation target.

10. The cell observation method according to Claim 8 or 9, wherein
the detection step includes a step of detecting a distribution of lipid in the observation target.

11. The cell observation method according to Claim 10, wherein
the determination step includes a step of counting the number of dead cells according to the number of cell nuclei distributed near clumps of lipid.

12. The cell observation method according to Claim 8 or 9, wherein
the detection step includes a step of generating a detection image that reflects detection of a distribution of protein in the observation target.

13. The cell observation method according to any one of Claims 4 to 12, wherein
the detection step includes a selection step of selecting a type of molecule to generate Raman-scattered light, according to a type of cell included in the observation target.

14. The cell observation method according to any one of Claims 4 to 13, wherein
the detection step includes a step of extracting a portion of the observation target as an observation target region and detecting Raman-scattered light in the observation target region.

15. The cell observation method according to Claim 14, wherein
the detection step includes a step of scanning a predetermined observation target region in the observation target with the first excitation light and the second excitation light, and detecting Raman-scattered light generated by the observation target region.

16. The cell observation method according to Claim 13 or 14, wherein
the detection step includes a step of displacing the observation target in a direction intersecting an irradiation direction of the first excitation light and the second excitation light, and detecting Raman-scattered light generated by a predetermined observation target region in the observation target.

17. The cell observation method according to Claim 7, wherein
the determination step includes a calculation step of performing image analysis on the detection image and calculating a ratio of at least one of live cells and dead cells.

18. The cell observation method according to Claim 17, wherein
the image analysis includes performing a Fourier transform on the detection image.

19. The cell observation method according to any one of Claims 4 to 18, wherein
the determination step further includes an output step of outputting a determination result to the outside, and
the output step includes a step of outputting a ratio of at least one of live cells and dead cells included in the observation target.

20. The cell observation method according to any one of Claims 4 to 19, wherein
the determination step includes a step of determining whether a cell is dead or alive based on a difference between detection images detected at different times for the same detection target.

21. The cell observation method according to any one of Claims 4 to 20, wherein
the determination step includes a step of determining whether a cell is dead or alive based on a distribution of shapes smaller than nuclei of cells appearing in a detection image.

22. The cell observation method according to any one of Claims 4 to 21, wherein
the determination step further includes a step of determining whether a dead cell included in a detection target died of apoptosis or died of necrosis.

23. The cell observation method according to any one of Claims 4 to 21, wherein
the determination step further includes a step of determining whether a dead cell included in a detection target died of apoptosis or died of necrosis, based on a distribution of shapes smaller than nuclei of cells appearing in a detection image.

24. The cell observation method according to any one of Claims 4 to 18, wherein
the determination step further includes an output step of outputting a determination result to the outside, and
the output step includes a step of outputting at least one of the number of live cells and the number of dead cells included in the observation target.

25. The cell observation method according to Claim 24, wherein
the output step includes a step of displaying whether each cell in the observation target is dead or alive.

26. The cell observation method according to any one of Claims 4 to 25, wherein
the determination step further includes an output step of outputting a determination result to the outside, and
the output step includes a step of sending a warning to the outside when at least one of the number of dead cells and a percentage of live cells in the observation target is greater than a predetermined threshold value.

27. The cell observation method according to any one of Claims 24 to 26, wherein
the output step includes outputting whether or not the observation target satisfies a predetermined quality standard.

28. The cell observation method according to any one of Claims 4 to 6, wherein
the detection step includes a step of generating a character sequence indicating a frequency distribution of the Raman-scattered light in the observation target.

29. The cell observation method according to any one of Claims 4 to 28, wherein
the detection step includes a first detection step of detecting a spectrum of Raman-scattered light generated from the observation target and a second detection step of detecting Raman-scattered light generated by irradiating with the first excitation light and the second excitation light corresponding to a predetermined region in a detection region of the spectrum detected in the first detection step.

30. The cell observation method according to Claim 29, wherein
the observation target includes a plurality of cell groups having a common point as a culture condition, and
the first detection step includes detecting a spectrum of Raman-scattered light generated from one cell group among the plurality of cell groups and the second detection step includes detecting Raman-scattered light generated by irradiating each of the other cell groups among the plurality of cell groups with the first excitation light and the second excitation light corresponding to a peak of the spectrum detected in the first detection step.

31. The cell observation method according to any one of Claims 4 to 30, wherein
the determination step includes determining that the observation target includes a dead cell when lipid droplets that have entered into cytoplasm or a cell nucleus are detected in the observation target.

32. The cell observation method according to any one of Claims 4 to 31, wherein
the detection step further includes an empty space detection step of detecting a refractive index distribution of the observation target and distinguishing empty spaces included in the observation target from intended detection targets in the observation target.

33. A cell observation apparatus comprising:
a detecting section that detects radiation light that is radiated from an observation target when the observation target is irradiated with irradiation light; and
a determining section that determines whether a cell included in the observation target is alive or dead based on the radiation light.

34. A cell observation apparatus comprising:
a first detecting section that detects radiation light that is radiated from an observation target when the observation target is irradiated with irradiation light; and
a second detecting section that detects the number of live cells included in the observation target based on the radiation light.

35. A cell observation apparatus comprising:
an acquiring section that acquires a spectrum of irradiation light that irradiates an observation target when the observation target is irradiated with the irradiation light; and
a detecting section that detects a state of at least one of lipid and protein included in the observation target based on the spectrum.

36. A cell observation apparatus comprising:
a detecting section that detects Raman-scattered light radiated from an observation target when the observation target is irradiated with first excitation light having a first wavelength and second excitation light having a second wavelength that has a wavelength difference relative to the wavelength of the first excitation light corresponding to normal mode oscillation of molecules included in a cell of the observation target; and
a determining section that determines whether a cell included in the observation target is dead or alive based on the Raman-scattered light detected by the detecting section.

37. A cell observation program that causes a calculator to:
detect Raman-scattered light radiated from an observation target when the observation target is irradiated with first excitation light having a first wavelength and second excitation light having a second wavelength that has a wavelength difference relative to the wavelength of the first excitation light corresponding to normal mode oscillation of molecules included in a cell of the observation target, and
determine whether a cell included in the observation target is dead or alive based on the detected Raman-scattered light.

38. A cell sheet manufacturing method comprising:
a preparation step of dividing cells to prepare a cell line;
a culture step of culturing the cell line in a cell sheet; and
an observation step of observing the cells using the cell observation method according to any one of Claims 1 to 32.

39. The cell sheet manufacturing method according to Claim 38, wherein
the observation step includes a determination step of determining pass/fail of the cell sheet.

40. A cell sheet manufacturing apparatus comprising:
a preparing section that divides cells to prepare a cell line;
a culturing section that cultures the cell line in a cell sheet; and
an observing section that observes the cells using the cell observation method according to any one of Claims 1 to 32.

41. The cell sheet manufacturing apparatus according to Claim 40, wherein the observing section includes a determining section that determines pass/fail of the cell sheet.
